# EUROPEAN PATENT APPLICATION

(11) **EP 4 682 822 A1**
(43) Date of publication of application: **21.01.2026**
(21) Application number: 25185770.2
(22) Date of filing: 27.06.2025
(51) Int. Cl.: G06T 7/00, A61B 5/026, A61B 5/145, A61B 5/00, G06T 7/11, G06T 5/94

(54) **IMAGE PROCESSING METHOD AND SYSTEM IN ANALYTE TESTING, MEDIUM, AND DEVICE**

(30) Priority: 16.07.2024 CN 202410951413
(71) Applicant: SenSura Pte. Ltd, 608526 Singapore (SG)
(72) Inventor: ZHENG, Hongzhi, Guangzhou, 510663 (CN)
(74) Representative: Metida

(57) **Abstract**

The present invention provides an image processing method and system in analyte testing, a medium, and a device. The method includes: obtaining an infrared grayscale image of a first area; converting the image into a two-dimensional matrix, performing derivation, and recording a quantity of pixel grayscale values that remain monotonous on both sides of the minimum value point; creating an equal-size matrix of a two-dimensional matrix, and performing non-zero substitution on the minimum value point; and using a matrix as a convolution kernel to convolute the created equal-size matrix, where a probability that a position corresponding to the point is in a target area is greater as a value of an element in the obtained convoluted matrix is greater, sequencing elements and corresponding positions thereof in the convolution matrix in descending order, and selecting the target area.

## Description

### TECHNICAL FIELD

The present invention relates to the field of optical analysis, and in particular to an image processing method and system in analyte testing, a medium, and a device.

### BACKGROUND

A fluorescence analysis method means a method for qualitative or quantitative analysis of fluorescence that can reflect characteristics of some substances and that is produced during a process in which the substances are in an excited state after being irradiated with ultraviolet light, and molecules in an excited state undergo a de-excitation process of collision and emission.

When blood glucose of the human body is tested in the fluorescence analysis method, an image collected includes an area at which a venous blood vessel is located and an area at which the venous blood vessel is not located, because a blood glucose content in the blood vessel and a blood glucose content in the skin are extremely different, if spectral data collection is directly performed on the image, a test result is inaccurate. Therefore, accurately finding a position of a blood vessel in the image is an important prerequisite for blood glucose testing in the fluorescence analysis, but none of existing testing technologies can accurately position the blood vessel.

Patent document US20160287147A1 discloses a device for non-invasive in vivo measurement using Raman spectroscopy, which uses Raman spectroscopy to measure blood glucose concentration in vivo. This solution has the following advantages: the solution has higher accuracy than the electrochemical method in US20100065441A1, but has the following disadvantage: a laboratory-level Raman spectroscopy system needs to be used for implementation and is bulky and expensive.

In addition, patent document CN118078277A discloses a non-invasive blood glucose testing method based on hyperspectral data analysis, which implements non-invasive testing. Absorption spectroscopy is used in the document. Spectral signals collected and analyzed include not only a spectral signal of blood glucose, but also a spectral signal of components such as skin tissue. Spectral signals of different wavelengths are mixed together, making it difficult to perform fine separation and extract a spectral signal related to blood glucose. In addition, factors such as differences in excitation light sources, human skin colors, and epidermal thicknesses also affect the intensity of the spectral signals, resulting in differences in the intensity of the spectral signal. A spectral signal ultimately collected is easily affected and cannot be strongly correlated with a blood glucose concentration, affecting accurate measurement of the blood glucose concentration. Similarly, a same problem also occurs in patent document CN108542402A.

### SUMMARY

To overcome defects in the prior art, the present invention is intended to provide an image processing method and system in analyte testing, a medium, and a device.

The image processing method in analyte testing according to the present invention includes:
image obtaining: obtaining an infrared grayscale image of a first area;
minimum value searching step: converting the infrared grayscale image into a two-dimensional matrix, performing derivation on each row and each column of the matrix in an x direction and a y direction in turn, searching for a minimum value point, and recording a quantity of pixel grayscale values that remain monotonous on both sides of the minimum value point;
equal-size matrix creating step: creating an equal-size matrix of a two-dimensional matrix, and performing non-zero substitution on the minimum value point; and
area selecting step: using a matrix as a convolution kernel to convolute the created equal-size matrix, where a probability that a position corresponding to the point is in a target area is greater as a value of an element in the obtained convoluted matrix is greater, sequencing elements and corresponding positions thereof in the convolution matrix in descending order, and selecting the target area.

Preferably, a distributed minimum value point drawn in the equal-size matrix is substituted with 1, and distributed minimum value points are substituted with 2 if the minimum value points coincide.

Preferably, the size of the matrix as the convolution kernel is (n/2)*(n/2), and n is a quantity of pixels whose grayscale values remain monotonous on both sides of the minimum value point.

The present invention further provides a method for testing an analyte, including the image processing method in analyte testing.

The present invention further provides an image processing system in analyte testing, including:
an image obtaining module, configured to obtain an infrared grayscale image of a first area;
a minimum value searching module, configured to convert the infrared grayscale image into a two-dimensional matrix, perform derivation on each row and each column of the matrix in an x direction and a y direction in turn, search for a minimum value point, and record a quantity of pixel grayscale values that remain monotonous on both sides of the minimum value point;
an equal-size matrix creation module, configured to create an equal-size matrix of a two-dimensional matrix, and perform non-zero substitution on the minimum value point; and
an area selection module, configured to use a matrix as a convolution kernel to convolute the created equal-size matrix, where a probability that a position corresponding to the point is in a target area is greater as a value of an element in the obtained convoluted matrix is greater, sequence elements and corresponding positions thereof in the convolution matrix in descending order, and select the target area.

Preferably, a distributed minimum value point drawn in the equal-size matrix is substituted with 1, and distributed minimum value points are substituted with 2 if the minimum value points coincide.

Preferably, the size of the matrix as the convolution kernel is (n/2)*(n/2), and n is a quantity of pixels whose grayscale values remain monotonous on both sides of the minimum value point.

The present invention further provides a system for testing an analyte, including the image processing system in analyte testing.

The present invention further provides a computer-readable storage medium that stores a computer program thereon, and when the computer program is executed by a processor, the steps of the image processing method in analyte testing are implemented.

The present invention further provides an electronic device, including a memory, a processor, and a computer program stored in the memory and runnable on the processor, where when the computer program is executed by the processor, the steps of the image processing method in analyte testing are implemented.

Compared with the conventional technologies, the present invention has the following beneficial effects.
1. In the technical solution of this application, the infrared light source is used as a blood vessel positioning light source. Because hemoglobin in human blood has strong absorption of infrared wavelength light waves, and a skin reflectivity at a blood vessel under the infrared light source collected by a detector is lower than a skin reflectivity in a skin area, and the skin does not absorb infrared light waves due to pigmentation in the skin such as age spots, which means that the method of selecting the blood vessel area based on a grayscale is not affected by skin quality and has good versatility.
2. In the technical solution of this application, the area at which the blood vessel is located on the skin can be accurately recognized, so that the accuracy of blood glucose testing on the human body can be improved through optical analysis.
3. Electrochemical reaction with the analyte is not required in the testing method of this application, and a testing manner is more easily implemented, especially when an analyte in a living body is tested, so that non-invasive testing can be achieved.
4. In a testing method in this application, spectral data of different areas can be obtained or stipulated in a protocol by utilizing uneven distribution of the analyte in the imaging area. Because other components in the imaging area except the analyte are distributed relatively uniformly, a difference in spectral data in different areas can directly demonstrate the information about the analyte (such as a concentration of the analyte) correlated to the spectral data after the influence of a non-analyte is excluded.
5. In the testing method in this application, fluorescence spectroscopy is used for testing, preventing a traditional Raman method for measuring the analyte, thereby achieving low costs and miniaturization of the testing system and achieving real-time testing.

### BRIEF DESCRIPTION OF DRAWINGS

Other features, objects, and advantages of the present invention will become more apparent by reading detailed description of non-limiting embodiments with reference to the following drawings:
FIG. 1 is a flow chart in a first embodiment;
FIG. 2 is a schematic diagram of a first image collected in a second embodiment;
FIG. 3 is a schematic diagram of a second image collected in the second embodiment;
FIG. 4 is a schematic diagram of a testing model in the second embodiment;
FIG. 5 is a schematic diagram of testing point-reference point spectral data obtained in the second embodiment;
FIG. 6 is an experimental result of analysis result accuracy of an analysis model;
FIG. 7 is a schematic structural diagram of an analyte testing device provided in a seventh embodiment;
FIG. 8 is a schematic structural diagram of an electronic device provided in an eighth embodiment;
FIG. 9 is a schematic structural diagram of an analyte testing watch provided in a seventh embodiment;
FIG. 10 is a schematic diagram of a back of the analyte testing watch;
FIG. 11 is an exploded view of the analyte testing watch;
FIG. 12 is a schematic diagram of a use state of the analyte testing watch;
FIG. 13 is a schematic diagram and cross-sectional diagram of distribution of blood vessels under an infrared light source; and
FIG. 14 is a flow chart of steps of an image processing method in analyte testing in a third embodiment.

In the accompanying drawings,
100: imaging area; 200: testing device;
201: light source; 202: imaging spectrum detection apparatus;
203: controller; 204: first bandpass filter;
205: lens; 206: second bandpass filter;
207: circuit board; 501: processor;
502: memory.

### DETAILED DESCRIPTION OF EMBODIMENTS

The present invention is described below in detail with reference to specific embodiments. The following embodiments help those skilled in the art further understand the present invention, but do not limit the present invention in any form. It should be noted that those skilled in the art may make some improvements and transformation without departing from the idea of the present invention. The improvements and transformation shall fall within the protection scope of the present invention.

### First embodiment

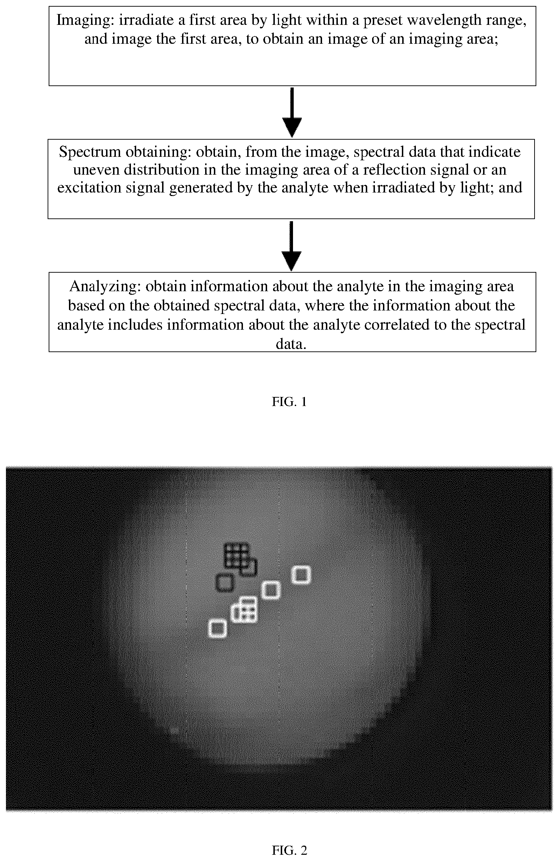

FIG. 1 is a flow chart of this embodiment. This embodiment provides a method for testing an analyte, including the following steps.

Imaging: providing light within a preset wavelength range through a light source to irradiate a first area, and imaging the first area through an imaging spectrum detection apparatus, to obtain an image of an imaging area. The image can demonstrate, during light irradiation within a preset wavelength range, distribution data and spectral data in the imaging area of a reflection signal or an excitation signal generated by the analyte when irradiated by light. The first area may be an area on a surface of a human skin. To prevent the influence of external light such as ambient light on testing, a collection window of the imaging spectrum detection apparatus needs to be tightly attached to the surface of the human skin in the first area, and the imaging area means an area within a lens range of the imaging spectrum detection apparatus. In general, the imaging area may be a part of the first area, or may be the same area as the first area.

Because obtaining the distribution data and spectral data of the analyte requires irradiation by light of different wavelength ranges, there are two implementation manners: the light is light with a larger wavelength range provided by one light source or light with smaller wavelength ranges provided by two light sources respectively. When there is one light source, the wavelength range of the light provided by the light source needs to cover both a wavelength range within which analyte distribution data can be obtained and a wavelength range within which analyte spectral data can be obtained. When there are two light sources, the two light sources provide different pieces of light. A wavelength range of the analyte distribution data can be obtained through a wavelength coverage of one piece of light, and a wavelength range of the analyte spectral data can be obtained through a wavelength coverage of the other piece of light. In addition, when there is one light source, one image is formed; when there are two light sources, two images are formed. For ease of processing, imaging areas of the two images are required to be the same, that is, the collection window of the imaging spectrum detection apparatus does not move on the surface of the human skin.

In this application, the analyte may be glucose, ketones, alcohol, lactate, oxygen, hemoglobin A1C, acetylcholine, amylase, bilirubin, cholesterol, chorionic gonadotropin, creatine kinase (such as CK-MB), creatine, creatinine, DNA, fructosamine, glutamine, growth hormone, hormone, peroxide, prostate-specific antigen, prothrombin, RNA, thyroid-stimulating hormone, or troponin in the blood vessel, or may be a drug such as antibiotics (such as gentamicin, vancomycin, or the like), digitoxin, digoxin, drugs of abuse, theophylline, or warfarin. In an implementation in which two or more analytes are tested, the analytes may be tested at the same or different time. In another embodiment, the analyte may alternatively be another substance on the surface of the human body, and non-invasive testing can be implemented according to the present invention.

Spectrum obtaining: obtaining, from the image by the imaging spectrum detection apparatus, spectral data that indicate uneven distribution in the imaging area of the reflection signal or the excitation signal generated by the analyte when irradiated by light. Specifically, the imaging area can be partitioned based on different pieces of distribution data, so that a position of the spectral data can be selected from different partitions.
analyzing step: obtaining information about the analyte in the imaging area based on the obtained spectral data, where the information about the analyte includes information about the analyte correlated to the spectral data. Due to a difference in distribution of the analyte in different areas, there is a difference in the reflection signal or excitation signal produced by the analytes when irradiated by light. For example, the human skin is divided into three parts: an epidermis tissue, a dermis tissue, and a subcutaneous tissue, and blood vessels such as veins are located in the subcutaneous tissue. Corresponding spectral data can be obtained by irradiating a skin area with blood vessels and a skin area without blood vessels irradiated by ultraviolet light, or the corresponding spectral data can be obtained from a skin area with thicker blood vessels and a skin area with thinner blood vessels. Therefore, a difference between the two pieces of spectral data can demonstrate the information about the analyte correlated to the spectral data in the blood vessels, such as intermediate information such as a degree of influence of the analyte on the spectral data for further analysis, or a concentration of the analyte and other information can be obtained directly through the analysis model.

### Second embodiment

This embodiment is based on the first embodiment. For example, glucose testing in human blood vessels is used as an example, and a non-invasive glucose testing method is provided, including the following steps.

Imaging: irradiating, by infrared light, skin on the wrist and the back of the hand at which the veins are located, in a first wavelength range of 800-1,000 nm, and collecting the first image of the imaging area, where the first wavelength range is preferably a near-infrared band; and irradiating, by ultraviolet light, a same position in a second wavelength range of 300-390 nm, to obtain the second image of the imaging area.

As shown in FIG. 2, an abscissa is a transverse coordinate of the first image, an ordinate is a longitudinal coordinate of the first image, white boxes represent selected testing point pixel blocks on venous blood vessels, and black boxes represent reference point pixel blocks on surrounding skin. In the first image, some of the infrared light penetrates the human skin and some is absorbed by the human skin, and is also absorbed by the venous blood vessels in large quantities in an area at which the venous blood vessels are located, presenting a characteristic that a pixel grayscale value of the area at which the venous blood vessels are located is small, and a pixel grayscale value of the area at which non-venous blood vessels are located is large, thereby easily dividing the imaging area into the area at which the venous blood vessels are located and the area at which the non-venous blood vessels are located.

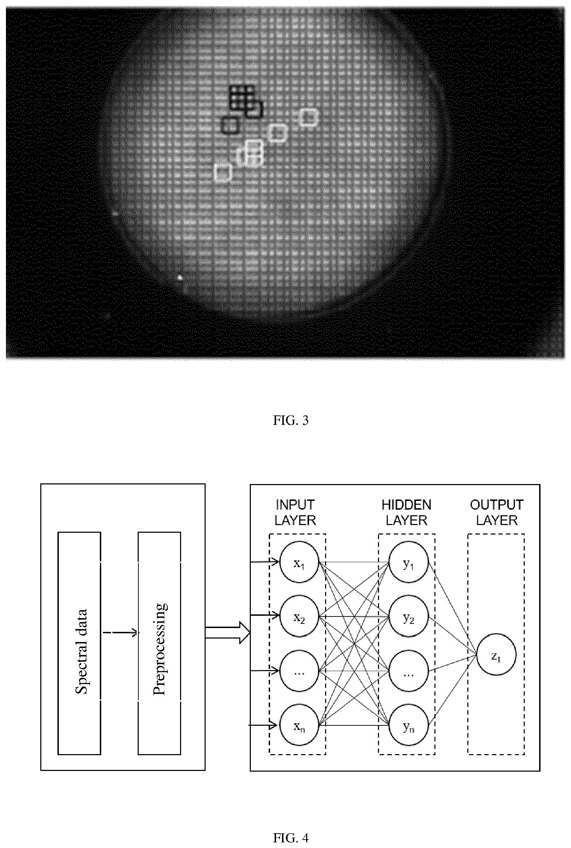

As shown in FIG. 3, an abscissa is a transverse coordinate of the second image, an ordinate is a longitudinal coordinate of the second image, white boxes represent selected testing point pixel blocks on the venous blood vessels, and black boxes represent reference point pixel blocks on surrounding skin. In the second image, because it is difficult to distinguish between the area at which the venous blood vessels are located and the area at which the non-venous blood vessels are located, the first image is required for distinguishing. Excitation light in the second wavelength range of 300-390 nm is used to obtain a high-quality effective fluorescence spectral signal. A main response band of the imaging spectrum detection apparatus is 400-800 nm. When the wavelength of the excitation light is less than 300 nm, a main peak of a fluorescence spectrum of the excited fluorescence radiation signal is in a band <400 nm, and it is difficult for the imaging spectrum detection apparatus to obtain the high-quality effective fluorescence spectral signal. When the wavelength of the excitation light used is >390 nm, the excitation light itself is visible light, the spectral signal of the excitation light and the fluorescence spectral signal are superimposed together, and it is difficult to eliminate the interference of the spectral signal of the excitation light and extract the effective fluorescence spectral signal. After absorbing ultraviolet light in the wavelength range of 300-390 nm, glucose in the venous blood vessels can emit a fluorescence radiation signal in a visible light band of 400-800 nm, and the band is in an effective response range of the imaging spectrum detection apparatus. A characteristic spectral intensity of the fluorescence radiation signal is positively correlated with the concentration of glucose, which has high fluorescence excitation efficiency.

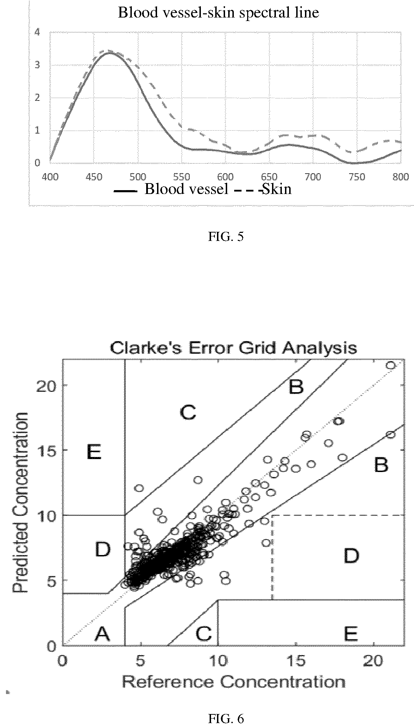

Spectral obtaining: based on grayscale distribution of pixels in the first image, dividing the imaging area into the area at which the venous blood vessels are located and the area at which the non-venous blood vessels are located, selecting a testing point from a position corresponding to the second image in the area at which the venous blood vessels are located, selecting a reference point from a position corresponding to the second image in the area at which the non-venous blood vessels are located, and respectively obtaining spectral data of the testing point and spectral data of the reference point in the second image. Specifically, based on a grayscale value of the pixel in the second image, a pixel whose grayscale value meets preset requirements is selected from the area at which the venous blood vessels are located as the testing point, or a combination of the pixel and an adjacent pixel is selected as the testing point, and a pixel whose grayscale value has a deviation from the selected testing point within a preset deviation range is selected from the area at which the non-venous blood vessels are located as the reference point, or a combination of the pixel and a plurality of adjacent pixels is selected as the reference point, and the fluorescence spectral data of the testing point and the fluorescence spectral data of the reference point are calculated in the second image. The spectral data is taken from the testing point, a pixel of the reference point, or a combined average of a plurality of pixels, which can be properly selected based on the width of the blood vessel. The combined average of the plurality of pixels can improve a signal-to-noise ratio, but is limited by the width of the blood vessel, preventing the spectral data from being taken from an extravascular area. Selection of a single pixel leads to high spatial resolution, which is proper for a thin blood vessel, but has a low signal-to-noise ratio. A preset requirement for the grayscale value may be using a point with the smallest grayscale value as the testing point, which is not limited in this application. A calculation result is shown in FIG. 5, an abscissa is the wavelength (in nm), an ordinate is a relative radiance (in W/nm), a solid line is the spectral data of the testing point, and a dashed line is the spectral data of the reference point. The grayscale value of the reference point and the grayscale value of the selected testing point are within the preset deviation range because the skin in the imaging area may be influenced by skin colors, pigmentation spots, cosmetics, and the like, which has a direct impact on the spectral data of the reference point, and the first image cannot distinguish the area including these influencing factors. These influencing factors can be effectively excluded by setting the preset deviation range of the grayscale value. In addition, the grayscale value and the grayscale value of the selected testing point are within the preset deviation range, which can ensure that the reference point is selected to be close to the testing point, such as an edge of the venous blood vessel. In this way, in addition to the blood vessels, the color, thickness, and other parameters of the epidermis tissue, the dermis tissue, and the subcutaneous tissues are approximately the same, so that a deviation between the spectral data of the testing point and the spectral data of the reference point can be excluded from the influence of a non-analyte as much as possible.

In addition to a spectrum reconstruction algorithm, the spectral data may alternatively be obtained by forming a radiometric calibration coefficient through previous radiometric calibration, and spectral lines are obtained by calculating the grayscale value * radiometric calibration coefficient.

When a combination of a plurality of pixels is selected at the testing point, the fluorescence spectral data at the testing point may be an average of fluorescence spectral data of these pixels. In addition, there may be one or more testing points and reference points. When there is a plurality of testing points and reference points, the average of the fluorescence spectral data of all testing points and the average of the fluorescence spectral data of all reference points can be calculated respectively.
analyzing step: preprocessing the obtained spectral data of the testing points and reference points, inputting the preprocessed data into a trained testing model, and outputting a concentration of glucose or an intermediate result of glucose correlated with the spectral data. When the testing model is trained, it is necessary to obtain spectral data and an accurate test result of a tested object, such as a blood collection test result, the spectral data is used as the input of the testing model, a blood collection test result is used as the output of the testing model, to train the testing model.

The testing model may be a convolutional neural network model, including an input layer, at least two convolutional layers, at least two activation function layers, a Flatten layer, a fully connected layer, and an output layer in sequence, and the convolutional layer and the activation function layer are spaced apart. An activation function used for the activation function layer is a Relu function.

In the convolutional neural network model, a size of each layer of convolutional kernels is 1, the number of convolutional kernels of a first convolutional layer is 32, and the number of convolutional kernels of a second layer is 64, which are both used to extract blood glucose features, and the output of the convolutional layer is transformed nonlinearly by the activation function. The Flatten layer flattens the output of the convolutional layer into a one-dimensional vector, to connect the subsequent fully connected layers, resulting in a final output dimension of 1. The Adam optimizer is used for model training during model training, a mean square error is used as a loss function, and a mean absolute error is calculated as a performance indicator of model evaluation.

When an output result of the testing model is glucose concentration, if an error between the output result and a measured standard glucose concentration value meets a preset condition, training is stopped, to obtain the testing model. When the output result of the testing model is the intermediate result of glucose correlated to the spectral data, such as an intermediate neuron result, if the error between the output result and the intermediate neuron result meets the preset condition, training is stopped to obtain the testing model. The intermediate neuron result is further model-corrected to obtain the concentration of glucose.

As shown in FIG. 4, the input layer is a spectral data input layer, which is obtained after the original spectral data is preprocessed. A hidden layer is a middle hidden layer, and a finally predicted blood glucose concentration value is output in an output layer after feature combination through convolution operation deep learning. Alternatively, through convolution operation deep learning, a neuron Output1 is output as an intermediate result value after feature combination, and model training is performed again on the intermediate result values Output1 and two infrared IR feature brightness values, to further correct a blood glucose prediction error, and the finally predicted blood glucose concentration value Output2 is output. Different parameters need to be set for a training degree of the testing model according to the needs, and a plurality of extracted glucose eigenvalues are continuously learned based on setting of different parameters, until an error between an output result and a standard glucose value of the above label value meets the requirements, and then training is stopped, to obtain the testing model.

Through a plurality of iterations of training, neurons can learn corresponding changes between different glucose concentrations and glucose spectral characteristics of different sampled objects, thereby improving the universality of the testing model and implementing prediction of glucose concentrations for different users.

In the whole glucose testing process, there is no need to pierce the skin for collecting blood or pierce the skin for implantation, and spectral information of a person to be tested is obtained based on the fluorescence spectrum, and a glucose test result of the person to be tested is obtained based on the spectral information, preventing pain and discomfort, and improving testing comfort and convenience. In the method, a spectral signal for blood vessels and a spectral signal for the skin can be finely distinguished, which provides a possibility for subsequent accurate extraction of a glucose signal, and also enables the spectral signal to be strongly correlated with the glucose concentration, implementing accurate measurement of the glucose concentration, so that a test result is more accurate, and processing is more easily performed.

FIG. 6 shows a schematic diagram of an experimental effect of the trained testing model, where an abscissa is a reference blood glucose concentration (in mmol/L) collected by a blood glucose meter, and an ordinate is a blood glucose concentration (in mmol/L) predicted in the method in the patent. There are totally 2,037 samples of tested objects, including 1,537 samples from a training set and 500 samples from a prediction set. Distribution of test results for the testing model can be learned from the figure, a MARD value of the predicted samples is 11.32%, and most of the samples fall in areas A and B, among which 87.03% of the samples fall in area A and 12.77% of the samples fall in area B, indicating that testing accuracy of the testing model is high.

### Third embodiment

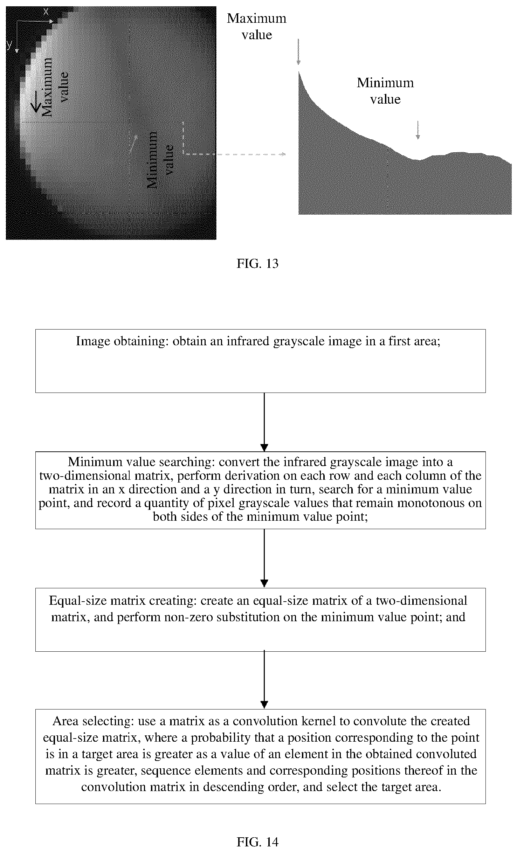

Based on the first embodiment and the second embodiment, this embodiment further describes an image processing method in analyte testing. As shown in FIG. 14, this embodiment provides an image processing method in analyte testing, including:
image obtaining: obtaining an infrared grayscale image in a first area;
minimum value searching: converting the infrared grayscale image into a two-dimensional matrix, performing derivation on each row and each column of the matrix in an x direction and a y direction in turn, searching for a minimum value point, and recording a quantity of pixel grayscale values that remain monotonous on both sides of the minimum value point;
equal-size matrix creation: creating an equal-size matrix of a two-dimensional matrix, and performing non-zero substitution on the minimum value point; and substituting a distributed minimum value point drawn in the equal-size matrix with 1, and substituting distributed minimum value points with 2 if the minimum value points coincide; and
area selection: using a matrix as a convolution kernel to convolute the created equal-size matrix, where a probability that a position corresponding to the point is in a target area is greater as a value of an element in the obtained convoluted matrix is greater, sequencing elements and corresponding positions thereof in the convolution matrix in descending order, and selecting the target area, where the size of the matrix as the convolution kernel is (n/2)*(n/2), and n is a quantity of pixels whose grayscale values remain monotonous on both sides of the minimum value point.

This embodiment further provides a method for testing an analyte, including the image processing method in analyte testing. This embodiment further provides a computer-readable storage medium that stores a computer program, and when the computer program is executed by a processor, the steps of the image processing method in analyte testing are implemented.

This embodiment further provides an electronic device, including a memory, a processor, and a computer program stored in the memory and runnable on the processor, where when the computer program is executed by the processor, the steps of the image processing method in analyte testing are implemented.

In an infrared grayscale image, a dark spot and a bright spot are distinguished based on a pixel gradient, where the dark spot means a blood vessel area and the bright spot means a skin area. First, a grayscale gradient of a field around each pixel in the sub-area is calculated for 2*2 periods, and then a peak value of the sub-area is found. A local maximum value (dark spot) and a local minimum value (bright spot), and 20 consecutive maximum values are selected as candidate positions of the dark spot. A specific blood vessel selection method includes the following steps.

### 1. Blood vessel positioning

An infrared light source with a central wavelength of 940 nm is selected as a blood vessel positioning light source. Because hemoglobin in human blood has strong absorption of infrared wavelength light waves, and a skin reflectivity at a blood vessel under the infrared light source collected by a detector is lower than a skin reflectivity in a skin area, and the skin does not absorb infrared light waves due to pigmentation in the skin such as age spots, which means that the method of selecting the blood vessel area based on a grayscale is not affected by skin quality and has good versatility.

As shown in FIG. 13, an infrared reflection intensity of the skin around the blood vessel varies uniformly along a cross-section direction of the blood vessel, and it is difficult to accurately delimit a specific blood vessel area. In addition, due to the uneven distribution of image illumination, the absorption of light energy by hemoglobin cannot be correctly reflected only by a grayscale strength, and a grayscale change trend in this direction needs to be considered. Therefore, the minimum value point can be used as the basis for determining the blood vessel. Ideally, assuming that the blood vessel is a standard cylinder, the center of the blood vessel absorbs infrared light the most, and a grayscale in the image is lower than a grayscale of a surrounding area. The image is regarded as a two-dimensional matrix, and derivation is performed on each row and each column of the matrix in the x direction and y direction in turn, and a common minimum value point in the x direction and y direction is the center of the blood vessel, a line of the minimum value point is a line of the center of the blood vessel, and a surrounding area of the blood vessel is the skin area. In FIG. 13, a figure on the left is a schematic diagram of skin with a blood vessel irradiated by the infrared light source, and a figure on the right is a schematic diagram of grayscale distribution at a cross-section.

There are still a lot of interference factors to be taken into account in actual collection, which means that the model needs to be optimized and a filter need to be added to eliminate interference.
a. The real blood vessel is not a standard cylinder, and under the influence of noise, minimum value points in the X direction and Y direction in the blood vessel area are not necessarily in a same position. Therefore, a distance between the minimum value points in the two directions can be close to each other during actual selection.
b. There is a large number of noise points, fine lines, and other interferences in the skin area in an actual image, and such interferences are also minimum value points in the image. Noise and fine lines are characterized by small areas and discrete distribution, and based on which, a design solution is as follows: (1) the blood vessel has a specific width, which is mathematically manifested as a monotonous area within a specific distance on both sides of the minimum value point, there is no other minimum value point, and therefore the noise and fine lines of a single pixel can be screened out accordingly; (2) the blood vessel area is a continuous area, minimum value points thereof are distributed relatively dense, and remaining noise can be filtered out accordingly.

The above solution is converted into an algorithm in the following steps: the infrared grayscale image is converted into a two-dimensional matrix, derivation is performed on each row and each column of the matrix in the x direction and y direction in turn, the minimum value point is searched for, and then a position is marked if grayscale values in n pixels on both sides of the minimum value point remain monotonous, where n depends on the width of the blood vessel, and as n is larger, a selected range is greater, and vice versa. The equal-size matrix of the two-dimensional matrix is created, non-zero substitution is performed on the minimum value point, and a distributed minimum value point drawn in the matrix is substituted with 1. Distributed minimum value points are substituted with 2 if the distributed minimum value points coincide. The created matrix is convoluted with a matrix of (n/2)*(n/2) (rounded down) as a convolution kernel. As a value of an element in the obtained convoluted matrix is greater, a probability that a position corresponding to the point is in a blood vessel area is greater, elements and corresponding positions thereof in the convolution matrix are sequenced in descending order, and the blood vessel area can be selected.

### 2. Skin positioning

For skin positioning, a point with a maximum brightness value near the blood vessel area is searched for. All selected coordinates of the blood vessel area are recorded, maximum values and minimum values of xₘₐₓ, xₘᵢₙ, yₘₐₓ, Ymin are selected, xₘₐₓ+m, xₘᵢₙ-m, yₘₐₓ+m, yₘᵢₙ₋m are used as searching area boundaries, where m is an expansion distance, which depends on an image spatial resolution. If the image spatial resolution is high, the value of m can be increased properly, and vice versa. Finally, an area within a range L of an image edge is filtered out, and L depends on a size of a possible color cast area of the lens edge. Finally obtained searching areas are sorted based on grayscales. As a grayscale is greater, an area is more possibly a skin area.

### 3. Selection of a skin pigment area

A 365 nm ultraviolet light source can reflect a skin pigmentation area well, and therefore collection can be performed on a same skin area by using the 365 nm ultraviolet light source after collection is performed by using an infrared light source. Coordinates of all previously selected blood vessel areas and skin areas are recorded, positions are marked in an ultraviolet image, and image grayscale values D of the positions are recorded. An average value Mu and a variance Sigma of the grayscale values are obtained separately. When |D-Mu|>Sigma×threshold, it means that the selected point is covered by the skin pigmentation area or a fluorescently labeled area, which can be filtered out. Left coordinates after filtering are selected available coordinates for the blood vessel area and the skin area.

Outlier point detection is performed on blood vessel and skin candidate dark spots, a threshold is set by using a standard deviation for filtering, and skin pigmentation, fluorescence, and other abnormal values are removed. A threshold is determined by | X - µ | ≤ T * σ, where µ is an average value of the dark spot or bright spot, σ is a standard deviation of the dark spot or bright spot, and T is used to set the threshold, which is usually a multiple of the standard deviation σ. The threshold of 1 is used as the basis for determining whether a selected data point is an outlier point (abnormal value), to obtain a more accurate coordinate position of the dark spot, that is, a coordinate position of a blood vessel.

The present invention further provides an image processing system in analyte testing. The image processing system in analyte testing can be implemented by performing flow steps of the image processing system in analyte testing. In other words, those skilled in the art can understand the image processing system in analyte testing as a preferred implementation of the image processing system in analyte testing.

### Fourth embodiment

This embodiment provides an image processing system in analyte testing, including:
an image obtaining module, configured to obtain an infrared grayscale image in a first area;
a minimum value searching module, configured to convert the infrared grayscale image into a two-dimensional matrix, perform derivation on each row and each column of the matrix in an x direction and a y direction in turn, search for a minimum value point, and record a quantity of pixel grayscale values that remain monotonous on both sides of the minimum value point;
an equal-size matrix creation module, configured to create an equal-size matrix of a two-dimensional matrix, and perform non-zero substitution on the minimum value point; and substitute a distributed minimum value point drawn in the equal-size matrix with 1, and substitute distributed minimum value points with 2 if the minimum value points coincide; and
an area selection module, configured to use a matrix as a convolution kernel to convolute the created equal-size matrix, where a probability that a position corresponding to the point is in a target area is greater as a value of an element in the obtained convoluted matrix is greater, sequence elements and corresponding positions thereof in the convolution matrix in descending order, and select the target area, where the size of the matrix as the convolution kernel is (n/2)*(n/2), and n is a quantity of pixels whose grayscale values remains monotonous on both sides of the minimum value point.

This embodiment further provides a system for testing an analyte, including the image processing system in analyte testing.

### Fifth embodiment

This embodiment is based on the second embodiment. Infrared light is replaced with visible light, and another non-invasive method for glucose testing is provided, including the following steps.

Imaging: collecting the first image of the imaging area by irradiating visible light on the skin, such as the wrist and the back of the hand, at which the veins are located; and irradiating, by ultraviolet light, a same position in a second wavelength range of 300-390 nm, to obtain the second image of the imaging area.

In the first image, due to the difference between a color of the area at which the venous blood vessels are located and a color of the area at which the non-venous blood vessels are located, the imaging area can be easily divided into the area at which the venous blood vessels are located and the area at which the non-venous blood vessels are located.

In the second image, because it is difficult to distinguish between the area at which the venous blood vessels are located and the area at which the non-venous blood vessels are located, the first image is required for distinguishing. Excitation light in the second wavelength range of 300-390 nm is used to obtain a high-quality effective fluorescence spectral signal. A main response band of the imaging spectrum detection apparatus is 400-800 nm. When the wavelength of the excitation light is less than 300 nm, a main peak of a fluorescence spectrum of the excited fluorescence radiation signal is in a band <400 nm, and it is difficult for the imaging spectrum detection apparatus to obtain the high-quality effective fluorescence spectral signal. When the wavelength of the excitation light used is >390 nm, the excitation light itself is visible light, the spectral signal of the excitation light and the fluorescence spectral signal are superimposed together, and it is difficult to eliminate the interference of the spectral signal of the excitation light and extract the effective fluorescence spectral signal. After absorbing ultraviolet light in the wavelength range of 300-390 nm, glucose in the venous blood vessels can emit a fluorescence radiation signal in a visible light band of 400-800 nm, and the band is in an effective response range of the imaging spectrum detection apparatus. A characteristic spectral intensity of the fluorescence radiation signal is positively correlated with the concentration of glucose, which has high fluorescence excitation efficiency.

Spectral obtaining: based on grayscale distribution of pixels in the first image, dividing the imaging area into the area at which the venous blood vessels are located and the area at which the non-venous blood vessels are located, selecting a testing point from the area at which the venous blood vessels are located, selecting a reference point from the area at which the non-venous blood vessels are located, and respectively obtaining spectral data of the testing point and spectral data of the reference point. Specifically, based on a grayscale value of the pixel in the second image, a pixel whose grayscale value meets preset requirements is selected from the area at which the venous blood vessels are located as the testing point, or a combination of the pixel and an adjacent pixel is selected as the testing point, and a pixel whose grayscale value has a deviation from the selected testing point within a preset deviation range is selected from the area at which the non-venous blood vessels are located as the reference point, or a combination of the pixel and a plurality of adjacent pixels is selected as the reference point, and the fluorescence spectral data of the testing point and the fluorescence spectral data of the reference point are calculated. The grayscale value of the reference point and the grayscale value of the selected testing point are within the preset deviation range because the skin in the imaging area may be influenced by skin colors, pigmentation spots, cosmetics, and the like, which has a direct impact on the spectral data of the reference point, and the first image cannot distinguish the area including all influencing factors. These influencing factors can be effectively excluded by setting the preset deviation range of the grayscale value.

When a combination of a plurality of pixels is selected at the testing point, the fluorescence spectral data at the testing point may be an average of fluorescence spectral data of these pixels. In addition, there may be one or more testing points and reference points. When there is a plurality of testing points and reference points, the average of the fluorescence spectral data of all testing points and the average of the fluorescence spectral data of all reference points can be calculated respectively.
analyzing step: preprocessing the obtained spectral data of the testing points and reference points, inputting the preprocessed data into a trained testing model, and outputting the concentration of glucose. When the testing model is trained, it is necessary to obtain spectral data and an accurate test result of a tested object, such as a blood collection test result, the spectral data is used as the input of the testing model, a blood collection test result is used as the output of the testing model, to train the testing model.

The testing model may be a convolutional neural network model, including an input layer, at least two convolutional layers, at least two activation function layers, a Flatten layer, a fully connected layer, and an output layer in sequence, and the convolutional layer and the activation function layer are spaced apart. An activation function used for the activation function layer is a Relu function.

In the convolutional neural network model, a size of each layer of convolutional kernels is 1, the number of convolutional kernels of a first convolutional layer is 32, and the number of convolutional kernels of a second layer is 64, which are both used to extract blood glucose features, and the output of the convolutional layer is transformed nonlinearly by the activation function. The Flatten layer flattens the output of the convolutional layer into a one-dimensional vector, to connect the subsequent fully connected layers, resulting in a final output dimension of 1. The Adam optimizer is used for model training during model training, a mean square error is used as a loss function, and a mean absolute error is calculated as a performance indicator of model evaluation.

If an error between the output result of the testing model and a standard glucose concentration meets a preset condition, training is stopped, to obtain the testing model.

Different parameters need to be set for a training degree of the testing model according to the needs, and a plurality of extracted glucose eigenvalues are continuously learned based on setting of different parameters, until an error between an output result and a standard glucose value of the above label value meets the requirements, and then training is stopped, to obtain the testing model.

Through a plurality of iterations of training, neurons can learn corresponding changes between different glucose concentrations and glucose spectral characteristics of different sampled objects, thereby improving the universality of the testing model and implementing prediction of glucose concentrations for different users.

In the whole glucose testing process, there is no need to collect blood or pierce the skin, and spectral information of a person to be tested is obtained based on the fluorescence spectrum, and a glucose test result of the person to be tested is obtained based on the spectral information, preventing pain and discomfort, and improving testing comfort and convenience. In the method, a spectral signal for blood vessels and a spectral signal for the skin can be finely distinguished, which provides a possibility for subsequent accurate extraction of a glucose signal, and also enables the spectral signal to be strongly correlated with the glucose concentration, implementing accurate measurement of the glucose concentration, so that a test result is more accurate, and processing is more easily performed.

### Sixth embodiment

This embodiment provides a system for testing an analyte. The system for testing an analyte can be implemented by performing flow steps of the method for testing an analyte. In other words, those skilled in the art can understand the method for testing an analyte as a preferred implementation of the system for testing an analyte. The system for testing an analyte includes the following modules.

An imaging module, configured to provide light within a preset wavelength range through a light source to irradiate a first area, and image the first area through an imaging spectrum detection apparatus, to obtain an image of an imaging area. The image can demonstrate, during light irradiation within a preset wavelength range, distribution data and spectral data in the imaging area of a reflection signal or an excitation signal generated by the analyte when irradiated by light. Because different wavelength ranges are required to obtain distribution data and spectral data of the analyte, the light may be either light corresponding to two wavelength ranges or light with a larger wavelength range that covers the two desired wavelength ranges. When there are two pieces of light, there are two obtained images. To facilitate processing, it is usually required that imaging areas of the two images be the same.

In this application, the analyte may be glucose, ketones, alcohol, lactate, oxygen, hemoglobin A1C, acetylcholine, amylase, bilirubin, cholesterol, chorionic gonadotropin, creatine kinase (such as CK-MB), creatine, creatinine, DNA, fructosamine, glutamine, growth hormone, hormone, peroxide, prostate-specific antigen, prothrombin, RNA, thyroid-stimulating hormone, or troponin in the blood vessel of an animal, or may be a drug such as antibiotics (such as gentamicin, vancomycin, or the like), digitoxin, digoxin, drugs of abuse, theophylline, and warfarin. In an implementation in which one or more analytes are tested, the analytes may be tested at the same or different time. In other embodiments, the analyte may alternatively be another substance in liquid.

A spectral obtaining module, configured to obtain, from the image by the imaging spectrum detection apparatus, spectral data that indicate uneven distribution in the imaging area of the reflection signal or the excitation signal generated by the analyte when irradiated by light. Specifically, the imaging area can be partitioned based on different pieces of distribution data, so that a position of the spectral data can be selected from different partitions.

An analysis module, configured to obtain information about the analyte in the imaging area based on the obtained spectral data, where the information about the analyte includes information about the analyte correlated to the spectral data. Due to a difference in distribution of the analyte in different areas, there is a difference in the reflection signal or excitation signal produced by the analytes when irradiated by light. With this feature, the difference between the two pieces of spectral data can be obtained, to accurately demonstrate the information about the analyte correlated to the spectral data, such as the concentration of the analyte.

Those skilled in the art know that, in addition to implementing the system provided in the present invention and each apparatus, module, and unit thereof in a purely computer-readable program code mode, the system provided in the present invention and each apparatus, module, and unit thereof can be enabled to implement same functions in the form of logic gates, switches, application-specific integrated circuits, programmable logic controllers, and embedded microcontrollers by performing logic programming of the method steps. Therefore, the system provided in the present invention and each apparatus, module, and unit thereof may be regarded as hardware components, and each apparatus, module, and unit thereof for implementing various functions may also be regarded as structures within the hardware components; and each apparatus, module, and unit thereof for implementing various functions may alternatively be regarded as software modules for implementing methods or structures within the hardware components.

### Seventh embodiment

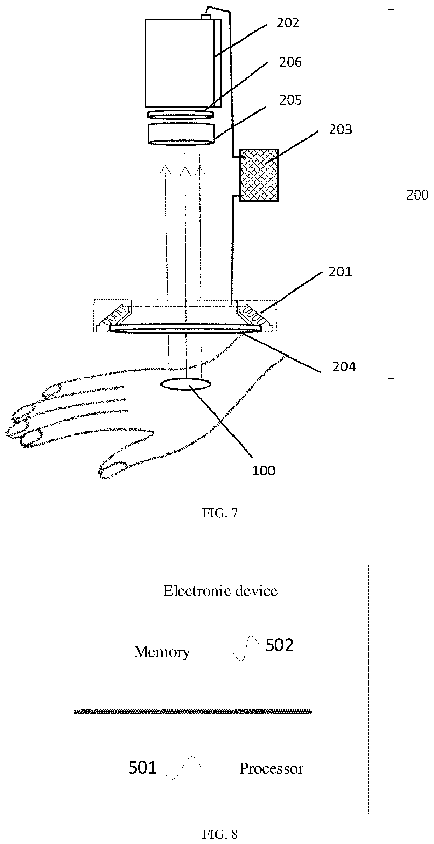

FIG. 7 shows an electronic device of this embodiment, which is specifically a testing device 200 of an analyte. The testing device 200 is a portable non-invasive testing device for a human body, which may be a single testing device or be integrated into a watch or mobile phone, to implement easy and rapid testing of the analyte in the surface of the human body.

The testing device 200 includes: a light source 201, an imaging spectrum detection apparatus 202, a controller 203, a first bandpass filter 204, a second bandpass filter 206, and a lens 205. The controller 203 establishes an electric connection or communication connection with the light source 201 and the imaging spectrum detection apparatus 202 separately.

The light source 201 can provide light within a preset wavelength range. Because obtaining the distribution data and spectral data of the analyte requires irradiation by light of different wavelength ranges, there are two implementation manners: a light source that can provide light with a larger wavelength range or two light sources that can respectively provide light with smaller wavelength ranges. When there is one light source, the wavelength range of the light provided by the light source needs to cover both a wavelength range within which analyte distribution data can be obtained and a wavelength range within which analyte spectral data can be obtained, such as a halogen lamp. When there are two light sources, the two light sources provide different pieces of light. A wavelength range of the analyte distribution data can be obtained through a wavelength coverage of one piece of light, and a wavelength range of the analyte spectral data can be obtained through a wavelength coverage of the other piece of light, such as an infrared lamp combined with an ultraviolet lamp, a visible light lamp combined with an ultraviolet lamp.

To enable the imaging area 100 to be irradiated evenly, a ring-shaped light source may be used. The light source has a plurality of light-emitting modules evenly distributed on a same circumference. When there are two light sources, the light-emitting modules of the two light sources are arranged alternately.

The imaging spectrum detection apparatus 202 can image the imaging area 100 based on an instruction to obtain a corresponding image, and can obtain corresponding spectral data based on the instruction. The imaging spectrum detection apparatus 202 includes a sensor and a periodic pixel-level light filtering structure arranged on a surface of the sensor. The periodic pixel-level light filtering structure is configured to perform spectral modulation on an incoming light signal, so that the sensor generates an image containing spectral information to be tested.

The periodic pixel-level light filtering structure includes a plurality of light filtering image element channels with pixel-level structures of different shapes. The plurality of light filtering image element channels have same specifications and sizes and are evenly arranged, and lengths and widths thereof are integer multiples of a pixel size in an image sensor. Light filtering image element channels of pixel-level light filtering structures of different shapes are corresponding to different spectral filter coefficients, and the pixel-level light filtering structures with different spectral filter coefficients are combined in a fixed order and then arranged periodically. The sensor modulates a received first tested light through the periodic pixel-level light filtering structure arranged on the surface of the sensor, to form a mosaic image containing spectral information, and then reconstructs the spectral data using an algorithm.

The controller 203 is configured to control the light source to provide light within a preset wavelength range to irradiate the first area, control the imaging spectrum detection apparatus to image the first area to obtain an image of an imaging area, and control the imaging spectrum detection apparatus to obtain, from the imaging area, spectral data that indicate uneven distribution in the imaging area of the reflection signal or excitation signal generated by the analyte when irradiated by light; and obtain information about the analyte in the imaging area based on the obtained spectral data, where the information about the analyte includes information about the analyte correlated to the spectral data. When there is one light source 201, one image is formed. When there are two light sources 201, two images are formed. When the first light source is turned on, the second light source is turned off. Similarly, when the second light source is turned on, the first light source is turned off. The two light sources do not interfere with each other.

The first bandpass filter 204 is located between the light source 201 and the imaging area 100. A function of the first bandpass filter 204 is to allow light within a preset wavelength range to pass through, while cutting off light outside the preset wavelength range, thereby reducing the impact of other external light on a test result.

The second bandpass filter 206 is located between the imaging spectrum detection apparatus 202 and the lens 205. A function of the second bandpass filter 206 is to allow light within a wavelength range in which the reflection signal or excitation signal generated by the analyte when irradiated by light is located to pass through, while cutting off light within another wavelength range, thereby reducing the influence of the reflection signal or excitation signal of a non-analyte on the test result.

The lens 205 can be configured to fix focus, to obtain a high-definition image. In another embodiment, the second bandpass filter 206 may alternatively be located on a side of the lens 205 away from the imaging spectrum detection apparatus 202, which is not limited in the present invention.

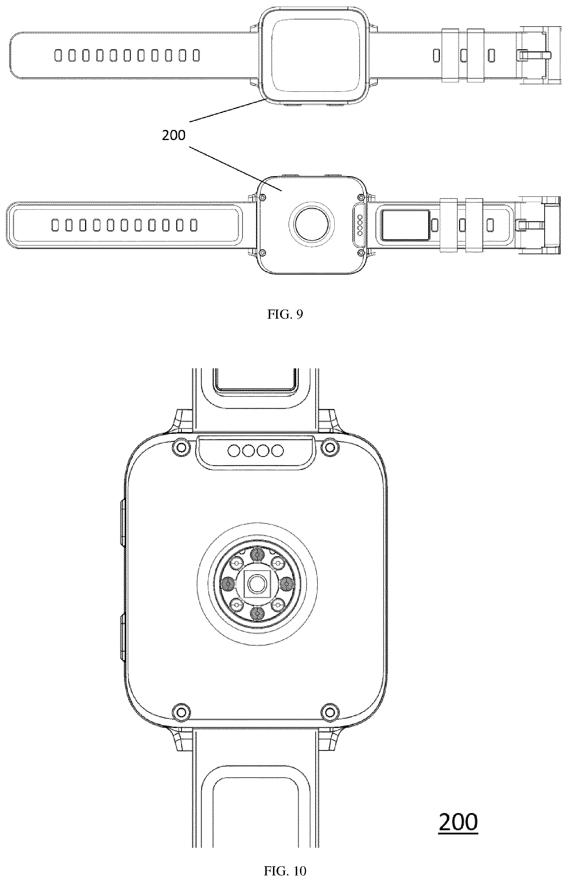

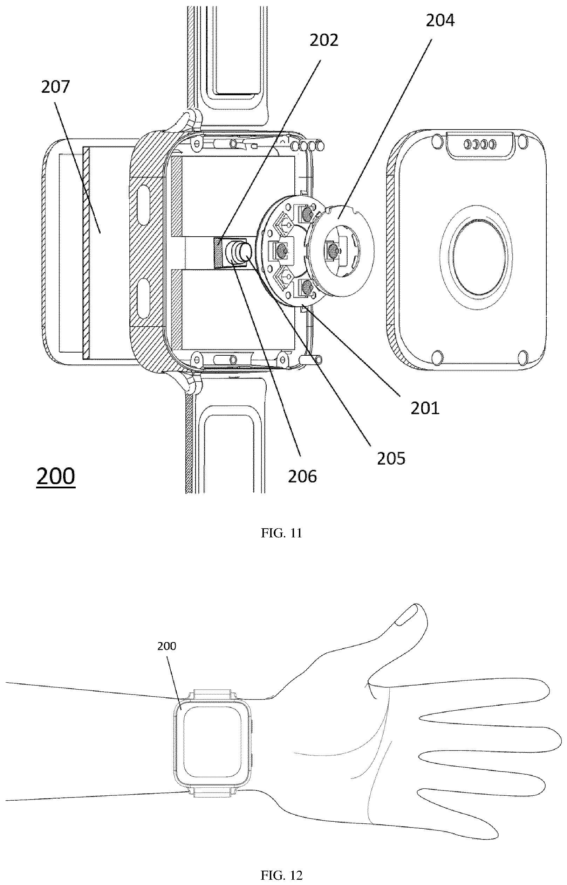

Based on the above description, FIG. 9 shows an analyte testing watch provided in this embodiment. A front of the watch is a display, and as shown in FIG. 10, a back of the watch has a light-transmitting window with a built-in testing device 200. As shown in FIG. 11, the light source 201 and the first bandpass filter 204 are both annular structures. Light-emitting modules of the light source 201 are distributed in a ring shape, and emitted light is filtered by the first bandpass filter 204, and then light with a required wavelength is output, and is irradiated onto a human body through the light-transmitting window on the back of the watch. The reflection signal or excitation signal of the human body enters the light-transmitting window, passes through the light source 201 and a hollow part in the middle of the first bandpass filter 204, enters a second bandpass filter 206 through a lens 205, and enters an imaging spectrum detection apparatus 202 after being filtered by the second bandpass filter 206. The imaging spectrum detection apparatus 202 is mounted on a circuit board 207, and a controller 203 (not shown in the figure) of the testing device 200 is also mounted on the circuit board 207. As shown in FIG. 12, to more accurately identify a position of a venous blood vessel, the watch can be worn on an inner side of the wrist.

### Eighth embodiment

FIG. 8 is a schematic structural diagram of an electronic device according to an embodiment of this application. As shown in FIG. 8, the electronic device includes at least one processor 501 and a memory 502 that is in communication connection with the at least one processor 501. The memory 502 stores instructions that can be executed by the at least one processor 501. The instructions are executed by the at least one processor 501, so that the at least one processor 501 can perform the above method for testing an analyte.

The memory 502 and the processor 501 are connected using a bus. The bus may include interconnected buses and bridges of any quantities. The bus connects various circuits of one or more processors 501 and memories 502. The bus may further connect a peripheral device, a voltage regulator, and various other circuits such as a power management circuit, which are all well known in the art and are not further described in the present invention. A bus interface provides an interface between the bus and a transceiver. The transceiver may be one component or a plurality of components, for example, a plurality of receivers and transmitters, to provide a unit that is configured to communicate with various other apparatuses on a transmission medium. Data processed by the processor 501 is transmitted on a wireless medium by using the antenna. Further, the antenna further receives data and transmits the data to the processor 501.

The processor 501 is responsible for managing the bus and general processing, and may further provide various functions, including timing, peripheral interfacing, voltage regulation, power management, and another control function. The memory 502 may be configured to store data used by the processor 501 when performing an operation.

The present invention also provides a computer-readable storage medium that stores a computer program, and when the computer program is executed by a processor, the above method for testing an analyte is implemented.

To be specific, those skilled in the art can understand that all or some of steps in the method of the above embodiments can be completed by instructing relevant hardware through a program. The program is stored in a storage medium and includes several instructions to enable a device (which may be a single-chip microcontroller, a chip, or the like) or a processor (processor) to perform all or some of the steps of the method described in various embodiments of this application. The above storage medium includes any medium that can store program code, such as a USB flash drive, a removable hard disk, a read-only memory (ROM, Read-Only Memory), a random access memory (RAM, Random Access Memory), a magnetic disk, or an optical disc.

Those skilled in the art can understand that the above implementations are specific embodiments for implementing the present invention, and in actual application, various changes may be made thereto in form and detail without departing from the spirit and scope of the present invention.

Specific embodiments of the present invention are described above. It should be understood that the present invention is not limited to the foregoing specific implementations. Those skilled in the art can make various variations or modifications within the scope of the claims, which does not affect the essence of the present invention. Embodiments in this application and the features in embodiments may be arbitrarily and mutually combined in the case of no conflict.

## Claims

1. An image processing method in analyte testing, comprising:
image obtaining: obtaining an infrared grayscale image of a first area;
minimum value searching step: converting the infrared grayscale image into a two-dimensional matrix, performing derivation on each row and each column of the matrix in an x direction and a y direction in turn, searching for a minimum value point, and recording a quantity of pixel grayscale values that remain monotonous on both sides of the minimum value point;
equal-size matrix creating step: creating an equal-size matrix of a two-dimensional matrix, and performing non-zero substitution on the minimum value point; and
area selecting step: using a matrix as a convolution kernel to convolute the created equal-size matrix, wherein a probability that a position corresponding to the point is in a target area is greater as a value of an element in the obtained convoluted matrix is greater, sequencing elements and corresponding positions thereof in the convolution matrix in descending order, and selecting the target area.

2. The image processing method in analyte testing according to claim 1, wherein a distributed minimum value point drawn in the equal-size matrix is substituted with 1, and distributed minimum value points are substituted with 2 if the minimum value points coincide.

3. The image processing method in analyte testing according to claim 1, wherein the size of the matrix as the convolution kernel is (n/2)*(n/2), and n is a quantity of pixels whose grayscale values remain monotonous on both sides of the minimum value point.

4. A method for testing an analyte, comprising the imaging process method in analyte testing according to any one of claims 1 to 3.

5. An image processing system in analyte testing, comprising:
an image obtaining module, configured to obtain an infrared grayscale image of a first area;
a minimum value searching module, configured to convert the infrared grayscale image into a two-dimensional matrix, perform derivation on each row and each column of the matrix in an x direction and a y direction in turn, search for a minimum value point, and record a quantity of pixel grayscale values that remain monotonous on both sides of the minimum value point;
an equal-size matrix creation module, configured to create an equal-size matrix of a two-dimensional matrix, and perform non-zero substitution on the minimum value point; and
an area selection module, configured to use a matrix as a convolution kernel to convolute the created equal-size matrix, wherein a probability that a position corresponding to the point is in a target area is greater as a value of an element in the obtained convoluted matrix is greater, sequence elements and corresponding positions thereof in the convolution matrix in descending order, and select the target area.

6. The image processing system in analyte testing according to claim 5, wherein a distributed minimum value point drawn in the equal-size matrix is substituted with 1, and distributed minimum value points are substituted with 2 if the minimum value points coincide.

7. The image processing system in analyte testing according to claim 5, wherein the size of the matrix as the convolution kernel is (n/2)*(n/2), and n is a quantity of pixels whose grayscale values remain monotonous on both sides of the minimum value point.

8. A system for testing an analyte, comprising the imaging process system in analyte testing according to any one of claims 5 to 7.

9. A computer-readable storage medium that stores a computer program thereon, wherein when the computer program is executed by a processor, the steps of the image processing method in analyte testing according to any one of claims 1 to 3 are implemented.

10. An electronic device, comprising a memory, a processor, and a computer program stored in the memory and runnable on the processor, wherein when the computer program is executed by the processor, the steps of the image processing method in analyte testing according to any one of claims 1 to 3 are implemented.
